# EUROPEAN PATENT APPLICATION

(11) **EP 4 702 935 A1**
(43) Date of publication of application: **04.03.2026**
(21) Application number: 25197774.0
(22) Date of filing: 25.08.2025
(51) Int. Cl.: A61B 10/00, A61B 10/02

(54) **DISPENSER INTEGRATED SAMPLE PREPARATION**

(30) Priority: 27.08.2024 US 202463687543 P
(71) Applicant: OP-Hygiene IP GmbH, 4704 Niederbipp (CH)
(72) Inventor: Ophardt, Heiner, Arisdorf (CH); Lang, Albrecht, Niederbipp (CH); Steltenkamp, Siegfried, Bonn (DE)
(74) Representative: Grünecker Patent- und Rechtsanwälte PartG mbB

(57) **Abstract**

A fluid dispenser including a fluid outlet for dispensing a fluid onto a user's hand, and a fluid collector for collecting at least some of the fluid after the fluid has contacted the user's hand. The fluid dispenser further includes a sample preparation mechanism for generating a sample from the fluid collected by the fluid collector, the sample being removable from the fluid dispenser for external analysis.

## Description

### Related Application

This application claims priority to the 27 August 2024 filing date of United States Provisional Patent Application Serial No. 63/687,543, which is incorporated herein by reference.

### Field of the Invention

This invention relates to hand cleaning fluid dispensers, and more generally to devices, systems and methods for collecting, analyzing, and identifying biological particles.

### Background of the Invention

Many diseases are caused by infectious agents such as viruses, bacteria, fungi, prions, and parasites. In order to combat the spread of these diseases, it is often useful to identify infected individuals. This can be accomplished, for example, by detecting and/or identifying the infectious biological particles that may be present in or on an individual's body.

The applicant has previously recognized that the detection of infectious agents can advantageously be performed using hand cleaning fluid dispensers, by collecting and analyzing the hand cleaning fluid after the fluid has contacted a user's hands (see United States Patent Application Publication No. 2022/0091011 to Steltenkamp et al., published 24 March 2022, and United States Patent Application Publication No. 2024/0099704 to Ophardt et al., published 28 March 2024, which are incorporated herein by reference). Hand cleaning fluid dispensers are widely available in many locations, including most washrooms and throughout many facilities such as hospitals and long term care homes, and are frequently used by many individuals. Using hand cleaning fluid dispensers to detect infectious agents can advantageously allow for the wide-spread screening or pre-screening of a large number of individuals for possible infections, including pre-symptomatic and asymptomatic individuals.

Hand cleaning fluid dispensers are well suited for performing analyses that are fast and relatively inexpensive, but which may produce results that have a relatively low accuracy and/or specificity.

### Summary of the Invention

To at least partially overcome some of the disadvantages of previously known systems, devices, and methods, in one aspect the present invention provides a fluid dispenser including a fluid outlet for dispensing a fluid onto a user's hand, and a fluid collector for collecting at least some of the fluid after the fluid has contacted the user's hand. The fluid dispenser further includes a sample preparation mechanism for generating a sample from the fluid collected by the fluid collector, the sample being removable from the fluid dispenser for external analysis.

The applicant has appreciated that it is often necessary and/or preferable to perform certain types of analyses in a laboratory setting using one or more analysis devices that are not incorporated into a hand cleaning fluid dispenser. For example, in some circumstances it may be desirable to obtain an image of a sample using a bulky and expensive device such as a scanning electron microscope, which may be impossible or impractical to incorporate into a hand cleaning fluid dispenser.

The applicant has appreciated that, by incorporating a sample preparation mechanism into a hand cleaning fluid dispenser, the effectiveness, efficiency, and speed of an external laboratory analysis can be improved. For example, the invention allows samples to be collected from individuals while they go about their normal daily activities (including hand washing), without requiring the individuals to visit a laboratory for sample collection.

The sample preparation mechanism preferably processes the fluid in order to prepare the sample for external analysis. For example, if the sample is being prepared for imaging by a scanning electron microscope, the fluid may be deposited onto a suitable substrate (such as a silicon wafer), dried, and coated with a suitable conductive material, such as gold. This preferably minimizes the amount of labor that needs to be performed by laboratory staff.

The fluid dispenser can also preferably be used to screen individuals to determine whether or not a sample should be prepared for external analysis. For example, the fluid dispenser may be equipped with one or more sensors that are capable of detecting signs of infection, such as an elevated body temperature. If a user of the dispenser is determined to have an infection or a possible infection, the dispenser is preferably configured to prepare a sample for external analysis using the fluid collected from the user. In some preferred embodiments, the dispenser may be configured to send a notification to laboratory staff when the sample is being prepared and/or is ready to be picked up for analysis.

In preferred embodiments of the invention, the dispenser associates each sample with the individual from which the sample was collected. For example, the fluid dispenser may be equipped with a facial recognition system for identifying users of the dispenser. Alternatively, the fluid dispenser may be configured to recognize an identification device carried by the user, such as an ID card, a key fob, a smart watch, and/or a smart phone. The identity of the user can be associated with the sample by, for example, adding a label to the sample, or by linking the user's identity to a label that is already present on the sample, such as a barcode. If the external analysis of the sample later reveals the presence of a concerning infection, the user is preferably contacted so that they can take additional steps, such as self-isolating and/or seeking medical attention.

In some preferred embodiments of the invention, the fluid dispenser may include an internal fluid analysis mechanism. Suitable internal fluid analysis mechanisms are disclosed, for example, in United States Patent Application Publication No. 2022/0091011 to Steltenkamp et al., published 24 March 2022; United States Patent Application Publication No. 2024/0099704 to Ophardt et al., published 28 March 2024; United States Patent No. 12,031,895 to Steltenkamp et al., issued 9 July 2024; and co-pending United States Patent Application Serial No. 18/669,881 to Ophardt et al., filed 21 May 2024, which are incorporated herein by reference.

The internal fluid analysis mechanism is preferably configured to perform a preliminary analysis of the fluid collected by the fluid collector. The internal fluid analysis mechanism may, for example, be configured to quantify the amount of biological particles that are present in the fluid, without necessarily providing a conclusive identification of the particles. For example, the internal fluid analysis mechanism may determine that the amount of bacteria-sized particles present in the fluid is greater than an expected background rate, indicating the possible presence of a bacterial infection. Upon determining the possible presence of an infection, the fluid dispenser is preferably configured to prepare a sample for external analysis, in order to more conclusively determine whether an infection is present, and the type of bacteria causing the infection.

Advantageously, the types of analyses that are performed internally in the dispenser and externally in the laboratory can be selected and adjusted according to the preferences, resources, and equipment of a given facility/organization. For example, in one facility the dispensers could be configured to prepare glass slides for external analysis in optical microscopes. In another facility, the dispensers could be provided with internal optical microscopes, and a preliminary optical analysis could be automatically performed within the dispensers themselves. On obtaining concerning or ambiguous results from the optical analysis, the dispensers could be configured to prepare samples for other types of external analysis, such as polymerase chain reaction (PCR), Raman spectroscopy, culturing of bacteria, hyperspectral imaging, fluorescence imaging, or scanning electron microscopy.

The applicant has appreciated that a dispenser in accordance with the invention can preferably provide very fast detection and analysis of infectious agents, including novel infectious agents that have not previously been identified or characterized. For example, an internal fluid analysis mechanism of the dispenser may detect concerning particles in the fluid collected from a user, triggering the preparation of one or more samples for external analysis. The external analysis may determine that the particles belong to a known class of bacteria, which has a low probability of causing disease, with the result that no further action is taken. Alternatively, the external analysis may determine that the particles are a known disease-causing bacteria, and steps can be taken to reduce the spread of the disease, including commencing treatment and self-isolation of the infected user. A further possibility is that the particles are found to be a novel bacteria, virus, prion, spore, parasite, or other biological entity that has not previously been characterized. In this case, the sample or samples can preferably be used to characterize the particle, including for example sequencing nucleic acid and/or amino acid sequences, obtaining images of the particle using various forms of microscopy, culturing the particle, and testing the effectiveness of various antibiotics. The external analysis can preferably be used to assess the disease causing potential of the particle, as well as the development of possible treatments such as antibiotics, antivirals, antifungals, and/or vaccines.

A fluid dispenser in accordance with the invention is preferably able to collect a specimen, analyze the specimen for signs of a possible infection, and prepare a sample for external analysis, all in a single location and without significant time delays. For example, if the dispenser determines that a user has a possible infection and that a laboratory analysis should be performed, the dispenser itself is able to prepare the sample using the fluid that has already been collected from the user. This avoids the need for laboratory staff to track down the user in order to collect a further sample for external analysis. As a result, the analysis can preferably proceed quickly, providing a significant advantage in the effort to stop or slow the spread of infectious diseases.

Further aspects of the invention include:
A fluid dispenser, which optionally incorporates any one or more features of any one or more of the preceding and/or following aspects, comprising: a fluid outlet for dispensing a fluid onto a user's hand; a fluid collector for collecting at least some of the fluid after the fluid has contacted the user's hand; and a sample preparation mechanism for generating a sample from the fluid collected by the fluid collector, the sample being removable from the fluid dispenser for external analysis.

A fluid dispenser, which optionally incorporates any one or more features of any one or more of the preceding and/or following aspects, wherein the sample preparation mechanism processes the fluid collected by the fluid collector to prepare the sample for the external analysis.

A fluid dispenser, which optionally incorporates any one or more features of any one or more of the preceding and/or following aspects, wherein the fluid dispensed from the fluid outlet is a hand cleaning fluid.

A fluid dispenser, which optionally incorporates any one or more features of any one or more of the preceding and/or following aspects, wherein the hand cleaning fluid contains alcohol.

A fluid dispenser, which optionally incorporates any one or more features of any one or more of the preceding and/or following aspects, wherein the sample preparation mechanism is configured to extract particles from the hand cleaning fluid and suspend the particles in a sample fluid.

A fluid dispenser, which optionally incorporates any one or more features of any one or more of the preceding and/or following aspects, wherein the sample fluid is alcohol-free.

A fluid dispenser, which optionally incorporates any one or more features of any one or more of the preceding and/or following aspects, wherein the sample fluid comprises a buffer fluid.

A fluid dispenser, which optionally incorporates any one or more features of any one or more of the preceding and/or following aspects, wherein the sample fluid comprises a cultivation broth.

A fluid dispenser, which optionally incorporates any one or more features of any one or more of the preceding and/or following aspects, wherein the sample fluid comprises a preservation fluid.

A fluid dispenser, which optionally incorporates any one or more features of any one or more of the preceding and/or following aspects, wherein the sample comprises a substrate and a specimen; wherein the specimen comprises at least some of the fluid collected by the fluid collector or a processed product generated from the fluid collected by the fluid collector.

A fluid dispenser, which optionally incorporates any one or more features of any one or more of the preceding and/or following aspects, wherein the sample preparation mechanism is configured to deposit the specimen onto the substrate.

A fluid dispenser, which optionally incorporates any one or more features of any one or more of the preceding and/or following aspects, wherein the sample preparation mechanism is configured to deliver the specimen into the substrate.

A fluid dispenser, which optionally incorporates any one or more features of any one or more of the preceding and/or following aspects, wherein the substrate comprises a microscope slide.

A fluid dispenser, which optionally incorporates any one or more features of any one or more of the preceding and/or following aspects, wherein the sample preparation mechanism is configured to cover the specimen deposited onto the microscope slide with a cover slip.

A fluid dispenser, which optionally incorporates any one or more features of any one or more of the preceding and/or following aspects, wherein the sample preparation mechanism is configured to dry the specimen deposited onto the substrate.

A fluid dispenser, which optionally incorporates any one or more features of any one or more of the preceding and/or following aspects, wherein the sample preparation mechanism is configured to apply a dye to at least one of: the specimen and the substrate.

A fluid dispenser, which optionally incorporates any one or more features of any one or more of the preceding and/or following aspects, wherein the sample preparation mechanism is configured to apply a sealing fluid to the specimen deposited onto the substrate.

A fluid dispenser, which optionally incorporates any one or more features of any one or more of the preceding and/or following aspects, wherein the substrate comprises a microfluidic chip.

A fluid dispenser, which optionally incorporates any one or more features of any one or more of the preceding and/or following aspects, wherein the substrate comprises a sample vial.

A fluid dispenser, which optionally incorporates any one or more features of any one or more of the preceding and/or following aspects, wherein the substrate comprises a silicon wafer.

A fluid dispenser, which optionally incorporates any one or more features of any one or more of the preceding and/or following aspects, wherein the substrate comprises a scanning electron microscopy mount or stub.

A fluid dispenser, which optionally incorporates any one or more features of any one or more of the preceding and/or following aspects, wherein the sample preparation mechanism is configured to coat the specimen in gold.

A fluid dispenser, which optionally incorporates any one or more features of any one or more of the preceding and/or following aspects, wherein the sample preparation mechanism is configured to perform at least one fixation step on the sample and/or the specimen.

A fluid dispenser, which optionally incorporates any one or more features of any one or more of the preceding and/or following aspects, wherein the at least one fixation step comprises applying a fixative fluid to the sample and/or the specimen.

A fluid dispenser, which optionally incorporates any one or more features of any one or more of the preceding and/or following aspects, wherein the sample preparation mechanism is configured to heat the sample and/or the specimen.

A fluid dispenser, which optionally incorporates any one or more features of any one or more of the preceding and/or following aspects, wherein the sample preparation mechanism is configured to cool the sample and/or the specimen.

A fluid dispenser, which optionally incorporates any one or more features of any one or more of the preceding and/or following aspects, wherein the sample preparation mechanism is configured to adjust a pH of the sample and/or the specimen.

A fluid dispenser, which optionally incorporates any one or more features of any one or more of the preceding and/or following aspects, wherein the sample preparation mechanism is configured to prepare the sample for analysis in a laboratory.

A fluid dispenser, which optionally incorporates any one or more features of any one or more of the preceding and/or following aspects, wherein the sample preparation mechanism is configured to prepare the sample for microscopy.

A fluid dispenser, which optionally incorporates any one or more features of any one or more of the preceding and/or following aspects, wherein the sample preparation mechanism is configured to prepare the sample for scanning electron microscopy.

A fluid dispenser, which optionally incorporates any one or more features of any one or more of the preceding and/or following aspects, wherein the sample preparation mechanism is configured to prepare the sample for Raman spectroscopy.

A fluid dispenser, which optionally incorporates any one or more features of any one or more of the preceding and/or following aspects, wherein the sample preparation mechanism is configured to prepare the sample for a polymerase chain reaction (PCR).

A fluid dispenser, which optionally incorporates any one or more features of any one or more of the preceding and/or following aspects, wherein the sample preparation mechanism is configured to prepare the sample for microbe culturing.

A fluid dispenser, which optionally incorporates any one or more features of any one or more of the preceding and/or following aspects, wherein the fluid dispenser comprises a sample discharge mechanism for discharging the sample from the fluid dispenser.

A fluid dispenser, which optionally incorporates any one or more features of any one or more of the preceding and/or following aspects, further comprising a dispenser housing.

A fluid dispenser, which optionally incorporates any one or more features of any one or more of the preceding and/or following aspects, wherein the sample discharge mechanism comprises a slot formed in the dispenser housing.

A fluid dispenser, which optionally incorporates any one or more features of any one or more of the preceding and/or following aspects, wherein the sample preparation mechanism is configured to generate the sample inside the dispenser housing.

A fluid dispenser, which optionally incorporates any one or more features of any one or more of the preceding and/or following aspects, wherein the sample discharge mechanism is configured to deliver the sample from inside of the dispenser housing to outside of the dispenser housing via the slot.

A fluid dispenser, which optionally incorporates any one or more features of any one or more of the preceding and/or following aspects, wherein the sample discharge mechanism comprises a storage container that is configured to store the sample.

A fluid dispenser, which optionally incorporates any one or more features of any one or more of the preceding and/or following aspects, wherein the storage container is accessible from outside of the fluid dispenser for collection of the sample.

A fluid dispenser, which optionally incorporates any one or more features of any one or more of the preceding and/or following aspects, wherein the storage container has a locking mechanism for preventing unauthorized access to the storage container.

A fluid dispenser, which optionally incorporates any one or more features of any one or more of the preceding and/or following aspects, wherein the fluid dispenser is configured to generate a plurality of the samples over time.

A fluid dispenser, which optionally incorporates any one or more features of any one or more of the preceding and/or following aspects, wherein the storage container is configured to store at least two of the plurality of the samples at a time.

A fluid dispenser, which optionally incorporates any one or more features of any one or more of the preceding and/or following aspects, wherein the storage container is configured to store more than one of the plurality of the samples at a time.

A fluid dispenser, which optionally incorporates any one or more features of any one or more of the preceding and/or following aspects, wherein the fluid dispenser is configured to label the sample.

A fluid dispenser, which optionally incorporates any one or more features of any one or more of the preceding and/or following aspects, wherein the sample preparation mechanism is configured to generate at least one of said samples every time the fluid dispenser is activated.

A fluid dispenser, which optionally incorporates any one or more features of any one or more of the preceding and/or following aspects, wherein the sample preparation mechanism is configured to generate the samples in accordance with a random sampling protocol.

A fluid dispenser, which optionally incorporates any one or more features of any one or more of the preceding and/or following aspects, wherein the sample preparation mechanism is configured to generate at least one of said samples when a specified condition is met.

A fluid dispenser, which optionally incorporates any one or more features of any one or more of the preceding and/or following aspects, further comprising a sample activation mechanism; wherein the sample preparation mechanism is configured to generate at least one of said samples when the sample activation mechanism is activated.

A fluid dispenser, which optionally incorporates any one or more features of any one or more of the preceding and/or following aspects, wherein the sample activation mechanism comprises a button that is manually activateable by a user of the fluid dispenser.

A fluid dispenser, which optionally incorporates any one or more features of any one or more of the preceding and/or following aspects, wherein the fluid dispenser comprises a user recognition system that is configured to recognize at least some users of the fluid dispenser; and wherein the sample preparation mechanism is configured to generate at least one of said samples when a specified user is operating the fluid dispenser.

A fluid dispenser, which optionally incorporates any one or more features of any one or more of the preceding and/or following aspects, wherein the user recognition system is configured to detect an identification device carried by the specified user.

A fluid dispenser, which optionally incorporates any one or more features of any one or more of the preceding and/or following aspects, wherein the user recognition system is configured to use facial recognition to identify the specified user.

A fluid dispenser, which optionally incorporates any one or more features of any one or more of the preceding and/or following aspects, wherein the sample preparation mechanism is configured to generate the samples in accordance with a sampling protocol.

A fluid dispenser, which optionally incorporates any one or more features of any one or more of the preceding and/or following aspects, further comprising a communication mechanism.

A fluid dispenser, which optionally incorporates any one or more features of any one or more of the preceding and/or following aspects, wherein the fluid dispenser is configured to modify the sampling protocol in accordance with instructions received via the communication mechanism.

A fluid dispenser, which optionally incorporates any one or more features of any one or more of the preceding and/or following aspects, wherein the fluid dispenser is configured to modify the sampling protocol in accordance with instructions received from an external laboratory via the communication mechanism.

A fluid dispenser, which optionally incorporates any one or more features of any one or more of the preceding and/or following aspects, further comprising a symptom detection mechanism.

A fluid dispenser, which optionally incorporates any one or more features of any one or more of the preceding and/or following aspects, wherein the symptom detection mechanism is configured to detect at least one symptom of a possible infection in a user of the fluid dispenser.

A fluid dispenser, which optionally incorporates any one or more features of any one or more of the preceding and/or following aspects, wherein the sample preparation mechanism is configured to generate at least one of said samples when the symptom detection mechanism detects that the user has the at least one symptom.

A fluid dispenser, which optionally incorporates any one or more features of any one or more of the preceding and/or following aspects, wherein the at least one symptom comprises an elevated body temperature.

A fluid dispenser, which optionally incorporates any one or more features of any one or more of the preceding and/or following aspects, wherein the symptom detection mechanism comprises a touchless thermometer.

A fluid dispenser, which optionally incorporates any one or more features of any one or more of the preceding and/or following aspects, wherein the at least one symptom comprises a cough.

A fluid dispenser, which optionally incorporates any one or more features of any one or more of the preceding and/or following aspects, wherein the symptom detection mechanism comprises a microphone.

A fluid dispenser, which optionally incorporates any one or more features of any one or more of the preceding and/or following aspects, further comprising a fluid analyzer for analyzing at least some of the fluid collected by the fluid collector.

A fluid dispenser, which optionally incorporates any one or more features of any one or more of the preceding and/or following aspects, wherein the fluid dispenser comprises at least one processor.

A fluid dispenser, which optionally incorporates any one or more features of any one or more of the preceding and/or following aspects, wherein the at least one processor is configured to determine whether or not to generate the sample.

A fluid dispenser, which optionally incorporates any one or more features of any one or more of the preceding and/or following aspects, wherein the at least one processor is configured to determine whether or not to generate the sample based, at least in part, on an internal analysis of the fluid performed by the fluid analyzer.

A fluid dispenser, which optionally incorporates any one or more features of any one or more of the preceding and/or following aspects, wherein the sample preparation mechanism is able to prepare different types of said samples.

A fluid dispenser, which optionally incorporates any one or more features of any one or more of the preceding and/or following aspects, wherein the different types of said samples are adapted for different types of said external analysis.

A fluid dispenser, which optionally incorporates any one or more features of any one or more of the preceding and/or following aspects, wherein the at least one processor is configured to determine which type of sample to prepare.

A fluid dispenser, which optionally incorporates any one or more features of any one or more of the preceding and/or following aspects, wherein the at least one processor is configured to determine which type of sample to prepare based, at least in part, on an internal analysis of the fluid performed by the fluid analyzer.

A fluid dispenser, which optionally incorporates any one or more features of any one or more of the preceding and/or following aspects, wherein the sample preparation mechanism is able to prepare more than one of said samples from each user of the fluid dispenser.

A fluid dispenser, which optionally incorporates any one or more features of any one or more of the preceding and/or following aspects, wherein the sample preparation mechanism is able to prepare more than one of said samples of different types from each user of the fluid dispenser.

A fluid dispenser, which optionally incorporates any one or more features of any one or more of the preceding and/or following aspects, wherein the sample preparation mechanism is configured to generate at least one of said samples when the fluid analyzer detects a specified property in the fluid collected by the fluid collector.

A fluid dispenser, which optionally incorporates any one or more features of any one or more of the preceding and/or following aspects, wherein the specified property comprises an elevated concentration of particles in the fluid.

A fluid dispenser, which optionally incorporates any one or more features of any one or more of the preceding and/or following aspects, wherein the particles comprise biological particles.

A fluid dispenser, which optionally incorporates any one or more features of any one or more of the preceding and/or following aspects, wherein the particles comprise bacteria.

A fluid dispenser, which optionally incorporates any one or more features of any one or more of the preceding and/or following aspects, wherein the particles comprise viral particles.

A fluid dispenser, which optionally incorporates any one or more features of any one or more of the preceding and/or following aspects, wherein the particles comprise spores.

A fluid dispenser, which optionally incorporates any one or more features of any one or more of the preceding and/or following aspects, wherein the fluid analyzer is configured to detect a presence of at least one type of particle in the fluid.

A fluid dispenser, which optionally incorporates any one or more features of any one or more of the preceding and/or following aspects, wherein the specified property comprises the presence of the at least one type of particle in the fluid.

A fluid dispenser, which optionally incorporates any one or more features of any one or more of the preceding and/or following aspects, wherein the fluid analyzer is configured to identify classes of particles present in the fluid.

A fluid dispenser, which optionally incorporates any one or more features of any one or more of the preceding and/or following aspects, wherein the specified property comprises a presence in the fluid of at least one specified class of particles.

A fluid dispenser, which optionally incorporates any one or more features of any one or more of the preceding and/or following aspects, wherein the specified property comprises an elevated concentration in the fluid of at least one specified class of particles.

A fluid dispenser, which optionally incorporates any one or more features of any one or more of the preceding and/or following aspects, wherein the fluid analyzer is configured to categorize the particles into at least one known class and at least one unknown class.

A fluid dispenser, which optionally incorporates any one or more features of any one or more of the preceding and/or following aspects, wherein the specified property comprises an elevated concentration in the fluid of the particles grouped into the at least one unknown class.

A fluid dispenser, which optionally incorporates any one or more features of any one or more of the preceding and/or following aspects, wherein the fluid dispenser is configured to provide a notification when the specified property is detected.

A fluid dispenser, which optionally incorporates any one or more features of any one or more of the preceding and/or following aspects, wherein the notification is transmitted to an external laboratory.

A fluid dispenser, which optionally incorporates any one or more features of any one or more of the preceding and/or following aspects, wherein the fluid dispenser is configured to provide a notification when the sample has been generated.

A fluid dispenser, which optionally incorporates any one or more features of any one or more of the preceding and/or following aspects, wherein the notification is transmitted to an external laboratory.

A fluid dispenser, which optionally incorporates any one or more features of any one or more of the preceding and/or following aspects, wherein the fluid dispenser is configured to record an association between the sample and the user from which the sample was generated.

A fluid dispenser, which optionally incorporates any one or more features of any one or more of the preceding and/or following aspects, wherein the fluid dispenser is configured to generate a label for the sample, the label identifying the user from which the sample was generated.

A fluid dispenser, which optionally incorporates any one or more features of any one or more of the preceding and/or following aspects, further comprising a laser that is used to generate the label on the sample.

A fluid dispenser, which optionally incorporates any one or more features of any one or more of the preceding and/or following aspects, wherein the samples are each provided with a unique label.

A fluid dispenser, which optionally incorporates any one or more features of any one or more of the preceding and/or following aspects, wherein, for each sample, the fluid dispenser is configured to record an association between the unique label and the user from which the sample was generated.

A fluid dispenser, which optionally incorporates any one or more features of any one or more of the preceding and/or following aspects, wherein the sample preparation mechanism is configured to separate particles present in the fluid by size.

A fluid dispenser, which optionally incorporates any one or more features of any one or more of the preceding and/or following aspects, wherein the sample preparation mechanism is configured to separate particles present in the fluid based on one or more particle characteristics.

A fluid dispenser, which optionally incorporates any one or more features of any one or more of the preceding and/or following aspects, wherein the sample preparation mechanism is configured to separate particles present in the fluid based on electrical properties of the particles.

A fluid dispenser, which optionally incorporates any one or more features of any one or more of the preceding and/or following aspects, wherein the sample preparation mechanism is configured to separate particles present in the fluid using a microfluidic particle sorter.

A fluid dispenser, which optionally incorporates any one or more features of any one or more of the preceding and/or following aspects, wherein the processing of the fluid collected by the fluid collector comprises dilution.

A fluid dispenser, which optionally incorporates any one or more features of any one or more of the preceding and/or following aspects, wherein the processing of the fluid collected by the fluid collector comprises adjusting concentration.

A fluid dispenser, which optionally incorporates any one or more features of any one or more of the preceding and/or following aspects, wherein the processing of the fluid collected by the fluid collector comprises extraction.

A fluid dispenser, which optionally incorporates any one or more features of any one or more of the preceding and/or following aspects, wherein the processing of the fluid collected by the fluid collector comprises purification.

A fluid dispenser, which optionally incorporates any one or more features of any one or more of the preceding and/or following aspects, wherein the processing of the fluid collected by the fluid collector comprises liquid exchange.

A fluid dispenser, which optionally incorporates any one or more features of any one or more of the preceding and/or following aspects, wherein the processing of the fluid collected by the fluid collector comprises adjusting pH.

A fluid dispenser, which optionally incorporates any one or more features of any one or more of the preceding and/or following aspects, wherein the processing of the fluid collected by the fluid collector comprises adjusting viscosity.

A fluid dispenser, which optionally incorporates any one or more features of any one or more of the preceding and/or following aspects, wherein the processing of the fluid collected by the fluid collector comprises adjusting one or more chemical, physical, and/or biological properties.

A fluid dispenser, which optionally incorporates any one or more features of any one or more of the preceding and/or following aspects, wherein the sample preparation mechanism is configured to deliver multiple specimens into or onto each of said substrates.

A fluid dispenser, which optionally incorporates any one or more features of any one or more of the preceding and/or following aspects, wherein the sample preparation mechanism is configured to prepare each of the multiple specimens for a different type of external analysis.

A fluid dispenser, which optionally incorporates any one or more features of any one or more of the preceding and/or following aspects, wherein the sample preparation mechanism is configured to prepare each of the multiple specimens from the fluid collected from a single user of the dispenser.

A fluid dispenser, which optionally incorporates any one or more features of any one or more of the preceding and/or following aspects, wherein the sample preparation mechanism is configured to prepare each of the multiple specimens from the fluid collected from a different user of the dispenser.

A fluid dispenser, which optionally incorporates any one or more features of any one or more of the preceding and/or following aspects, wherein each of the multiple specimens are physically separated in or on the substrate.

A fluid dispenser, which optionally incorporates any one or more features of any one or more of the preceding and/or following aspects, wherein each of the multiple specimens contain different types of particles derived from the fluid collected by the fluid collector.

A fluid dispenser, which optionally incorporates any one or more features of any one or more of the preceding and/or following aspects, wherein each of the multiple specimens contain particles of different sizes.

A system, which optionally incorporates any one or more features of any one or more of the preceding and/or following aspects, comprising: a fluid dispenser; and an analysis device that is external to the fluid dispenser; wherein the fluid dispenser comprises: a fluid outlet for dispensing a fluid onto a user's hand; a fluid collector for collecting at least some of the fluid after the fluid has contacted the user's hand; and a sample preparation mechanism for generating a sample from the fluid collected by the fluid collector, the sample being removable from the fluid dispenser for analysis by the analysis device.

A system, which optionally incorporates any one or more features of any one or more of the preceding and/or following aspects, wherein the fluid dispenser comprises the fluid dispenser in accordance with any one or more of the preceding aspects.

A system, which optionally incorporates any one or more features of any one or more of the preceding and/or following aspects, further comprising a delivery mechanism for delivering the sample from the fluid dispenser to a location of the analysis device.

A system, which optionally incorporates any one or more features of any one or more of the preceding and/or following aspects, wherein the delivery mechanism comprises a robot that is configured to retrieve the sample from the fluid dispenser and delivery the sample to the location of the analysis device.

A system, which optionally incorporates any one or more features of any one or more of the preceding and/or following aspects, wherein the delivery mechanism comprises a pneumatic conveying system.

A system, which optionally incorporates any one or more features of any one or more of the preceding and/or following aspects, wherein the fluid dispenser is configured to receive results from the analysis performed by the analysis device.

A system, which optionally incorporates any one or more features of any one or more of the preceding and/or following aspects, wherein the fluid dispenser is configured to modify the sampling protocol based, at least in part, on the results received from the analysis performed by the analysis device.

A system, which optionally incorporates any one or more features of any one or more of the preceding and/or following aspects, wherein the fluid dispenser is configured to modify an internal particle recognition system based, at least in part, on the results received from the analysis performed by the analysis device.

A system, which optionally incorporates any one or more features of any one or more of the preceding and/or following aspects, wherein the system comprises a communication network.

A system, which optionally incorporates any one or more features of any one or more of the preceding and/or following aspects, wherein the communication network is configured to provide a notification based, at least in part, on the results of the analysis performed by the analysis device.

A system, which optionally incorporates any one or more features of any one or more of the preceding and/or following aspects, wherein the notification is transmitted to the user from which the sample was generated.

A system, which optionally incorporates any one or more features of any one or more of the preceding and/or following aspects, wherein the notification is transmitted to a public health authority.

A system, which optionally incorporates any one or more features of any one or more of the preceding and/or following aspects, wherein the notification is transmitted to an administrator of a facility where the fluid dispenser is located.

A method, which optionally incorporates any one or more features of any one or more of the preceding and/or following aspects, comprising: using a fluid dispenser to dispense fluid onto a user's hand; using the fluid dispenser to collect at least some of the fluid after the fluid has contacted the user's hand; and using the fluid dispenser to prepare a sample for external analysis, the sample being derived from the fluid collected after having contacted the user's hand.

A method, which optionally incorporates any one or more features of any one or more of the preceding and/or following aspects, wherein the fluid dispenser comprises the fluid dispenser in accordance with any one or more of the preceding aspects.

A method, which optionally incorporates any one or more features of any one or more of the preceding and/or following aspects, further comprising: delivering the sample to an external analysis device.

A method, which optionally incorporates any one or more features of any one or more of the preceding and/or following aspects, further comprising: analyzing the sample using the external analysis device.

A fluid dispenser, which optionally incorporates any one or more features of any one or more of the preceding and/or following aspects, wherein the substrate comprises a polymer substrate, a metal substrate, a glass substrate, and/or a silicon substrate.

A fluid dispenser, which optionally incorporates any one or more features of any one or more of the preceding and/or following aspects, wherein the substrate comprises a silicon wafer, a metal wafer, and/or a glass wafer.

A fluid dispenser, which optionally incorporates any one or more features of any one or more of the preceding and/or following aspects, wherein the sample preparation mechanism is configured to coat the specimen in metal.

A fluid dispenser, which optionally incorporates any one or more features of any one or more of the preceding and/or following aspects, wherein the sample preparation mechanism is configured to coat the specimen in a polymer.

A fluid dispenser, which optionally incorporates any one or more features of any one or more of the preceding and/or following aspects, wherein the sample preparation mechanism is configured to prepare the sample for hyperspectral imaging.

A fluid dispenser, which optionally incorporates any one or more features of any one or more of the preceding and/or following aspects, wherein the sample preparation mechanism is configured to prepare the sample for fluorescence imaging.

A fluid dispenser, which optionally incorporates any one or more features of any one or more of the preceding and/or following aspects, wherein the symptom detection mechanism detects any suitable body parameter, including heart rate, blood oxygen level, and/or blood pressure.

A fluid dispenser, which optionally incorporates any one or more features of any one or more of the preceding and/or following aspects, wherein the symptom detection mechanism communicates with a monitoring device worn by the user.

A fluid dispenser, which optionally incorporates any one or more features of any one or more of the preceding and/or following aspects, wherein the monitoring device comprises a smart watch.

A fluid dispenser, which optionally incorporates any one or more features of any one or more of the preceding and/or following aspects, wherein the particles comprise pathogenic particles.

A fluid dispenser, which optionally incorporates any one or more features of any one or more of the preceding and/or following aspects, wherein the particles comprise one or more of: bacteria, fungi, virus, poison, and/or radiation.

A fluid dispenser, which optionally incorporates any one or more features of any one or more of the preceding and/or following aspects, comprising: a fluid outlet for dispensing a fluid onto a user's hand; a fluid collector for collecting at least some of the fluid after the fluid has contacted the user's hand; and a sample preparation mechanism for generating a sample from the fluid collected by the fluid collector, the sample being removable from the fluid dispenser for external analysis.

A fluid dispenser, which optionally incorporates any one or more features of any one or more of the preceding and/or following aspects, wherein the sample preparation mechanism processes the fluid collected by the fluid collector to prepare the sample for the external analysis.

A fluid dispenser, which optionally incorporates any one or more features of any one or more of the preceding and/or following aspects, wherein the fluid dispensed from the fluid outlet is a hand cleaning fluid.

A fluid dispenser, which optionally incorporates any one or more features of any one or more of the preceding and/or following aspects, wherein the sample preparation mechanism is configured to extract particles from the hand cleaning fluid and suspend the particles in a sample fluid.

A fluid dispenser, which optionally incorporates any one or more features of any one or more of the preceding and/or following aspects, wherein the sample fluid comprises at least one of: a buffer fluid, a cultivation broth, and a preservation fluid.

A fluid dispenser, which optionally incorporates any one or more features of any one or more of the preceding and/or following aspects, wherein the sample comprises a substrate and a specimen; wherein the specimen comprises at least some of the fluid collected by the fluid collector or a processed product generated from the fluid collected by the fluid collector; and wherein the sample preparation mechanism is configured to: deposit the specimen onto the substrate and/or deliver the specimen into the substrate.

A fluid dispenser, which optionally incorporates any one or more features of any one or more of the preceding and/or following aspects, wherein the substrate comprises at least one of: a microscope slide, a microfluidic chip, a sample vial, a silicon wafer, and a scanning electron microscopy mount or stub.

A fluid dispenser, which optionally incorporates any one or more features of any one or more of the preceding and/or following aspects, wherein the sample preparation mechanism is configured to at least one of: dry the specimen deposited onto the substrate; apply a dye to at least one of: the specimen and the substrate; apply a sealing fluid to the specimen deposited onto the substrate; coat the specimen in gold; perform at least one fixation step on the sample and/or the specimen; apply a fixative fluid to the sample and/or the specimen; heat the sample and/or the specimen; cool the sample and/or the specimen; adjust a pH of the sample and/or the specimen; prepare the sample for analysis in a laboratory; prepare the sample for microscopy; prepare the sample for scanning electron microscopy; prepare the sample for Raman spectroscopy; prepare the sample for a polymerase chain reaction (PCR); prepare the sample for microbe culturing; coat the specimen in metal; coat the specimen in a polymer; prepare the sample for hyperspectral imaging; prepare the sample for fluorescence imaging; separate particles present in the fluid by size; separate particles present in the fluid based on one or more particle characteristics; separate particles present in the fluid based on electrical properties of the particles; separate particles present in the fluid using a microfluidic particle sorter; dilute the fluid; adjust a concentration of the fluid; extract a component from the fluid; purify the specimen; perform a liquid exchange; adjust a viscosity of the fluid; and adjust one or more chemical, physical, and/or biological properties of the sample.

A fluid dispenser, which optionally incorporates any one or more features of any one or more of the preceding and/or following aspects, further comprising a dispenser housing; wherein the sample preparation mechanism is configured to generate the sample inside the dispenser housing; and wherein the fluid dispenser comprises a sample discharge mechanism for discharging the sample from inside of the dispenser housing to outside of the dispenser housing.

A fluid dispenser, which optionally incorporates any one or more features of any one or more of the preceding and/or following aspects, further comprising a storage container that is configured to store the sample; and wherein the storage container is accessible from outside of the fluid dispenser for collection of the sample.

A fluid dispenser, which optionally incorporates any one or more features of any one or more of the preceding and/or following aspects, wherein the fluid dispenser is configured to label the sample.

A fluid dispenser, which optionally incorporates any one or more features of any one or more of the preceding and/or following aspects, further comprising a symptom detection mechanism; wherein the symptom detection mechanism is configured to detect at least one symptom of a possible infection in a user of the fluid dispenser; and wherein the sample preparation mechanism is configured to generate the sample if the symptom detection mechanism detects that the user has the at least one symptom.

A fluid dispenser, which optionally incorporates any one or more features of any one or more of the preceding and/or following aspects, further comprising: a fluid analyzer for analyzing at least some of the fluid collected by the fluid collector; and at least one processor; wherein the at least one processor is configured to determine whether or not to generate the sample based, at least in part, on an internal analysis of the fluid performed by the fluid analyzer.

A fluid dispenser, which optionally incorporates any one or more features of any one or more of the preceding and/or following aspects, wherein the sample preparation mechanism is able to prepare different sample types adapted for different external analyses; wherein the at least one processor is configured to determine which sample type to prepare based, at least in part, on the internal analysis of the fluid performed by the fluid analyzer.

A fluid dispenser, which optionally incorporates any one or more features of any one or more of the preceding and/or following aspects, wherein the fluid dispenser is configured to provide a notification when the sample has been generated; and wherein the fluid dispenser is configured to transmit the notification to an external laboratory.

### Brief Description of the Drawings

Further aspects and advantages of the invention will appear from the following description taken together with the accompanying drawings, in which:
Figure 1 is a perspective view of a hand cleaning fluid dispenser in accordance with a first embodiment of the present invention;
Figure 2 is a schematic front view of the hand cleaning fluid dispenser shown in Figure 1 and an external analysis device;
Figure 3 is a front view of the hand cleaning fluid dispenser shown in Figure 1 dispensing hand cleaning fluid onto a user's hand;
Figure 4 is a perspective view of the hand cleaning fluid dispenser shown in Figure 1, with a sample shown being discharged from the dispenser;
Figure 5 is a partial internal view of the hand cleaning fluid dispenser shown in Figure 1, showing a sample preparation mechanism of the dispenser;
Figure 6 is a partial internal view of the hand cleaning fluid dispenser shown in Figure 1, showing the sample preparation mechanism depositing a drop of a sample fluid onto a glass slide;
Figure 7 is a partial internal view of the hand cleaning fluid dispenser shown in Figure 1, showing the drop of sample fluid on the glass slide;
Figure 8 is a partial internal view of the hand cleaning fluid dispenser shown in Figure 1, showing the glass slide after the sample fluid has been dried;
Figure 9 is a partial internal view of the hand cleaning fluid dispenser shown in Figure 1, showing the glass slide being discharged from the dispenser;
Figure 10 is a partial internal view of the hand cleaning fluid dispenser shown in Figure 1, showing a first alternative construction of the sample preparation mechanism;
Figure 11 is a partial internal view of the hand cleaning fluid dispenser shown in Figure 1, showing a second alternative construction of the sample preparation mechanism;
Figure 12 is a partial internal view of the hand cleaning fluid dispenser shown in Figure 1, showing a third alternative construction of the sample preparation mechanism;
Figure 13 is a schematic representation of an internal fluid processing channel of the hand cleaning fluid dispenser shown in Figure 1;
Figure 14 is a perspective view of a microfluidic chip sample that may be prepared by the hand cleaning fluid dispenser shown in Figure 1;
Figure 15 is a perspective view of a hand cleaning fluid dispenser in accordance with a second embodiment of the present invention;
Figure 16 is a perspective view of the hand cleaning fluid dispenser shown in Figure 15, showing a sample container of the dispenser in an open condition;
Figure 17 is a perspective view of the hand cleaning fluid dispenser shown in Figure 15, showing a robot retrieving samples from the open sample container; and
Figure 18 is a perspective view of a hand cleaning fluid dispenser in accordance with a third embodiment of the present invention.

### Detailed Description of the Drawings

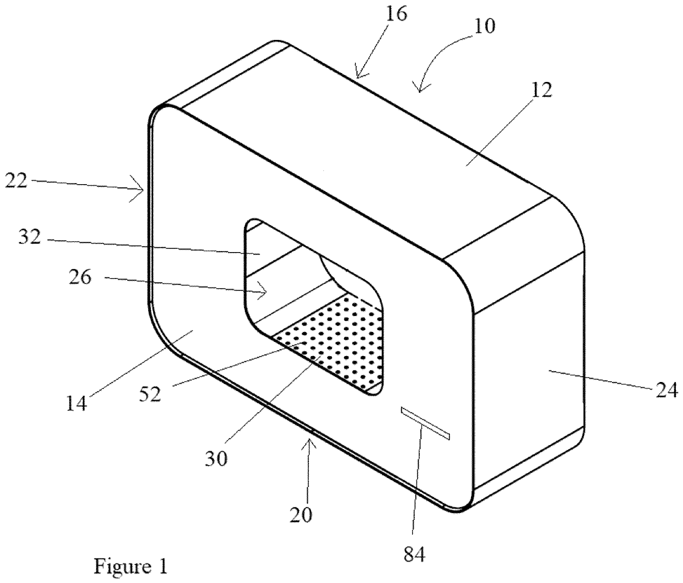

Figures 1 to 4 show a hand cleaning fluid dispenser 10 in accordance with a first embodiment of the present invention. The fluid dispenser 10 has a generally rectangular housing 12 with a front wall 14, a back wall 16, a top wall 18, a bottom wall 20, a right side wall 22, and a left side wall 24. The housing 12 has a forwardly open chamber 26 that extends rearwardly from the center of the front wall 14.

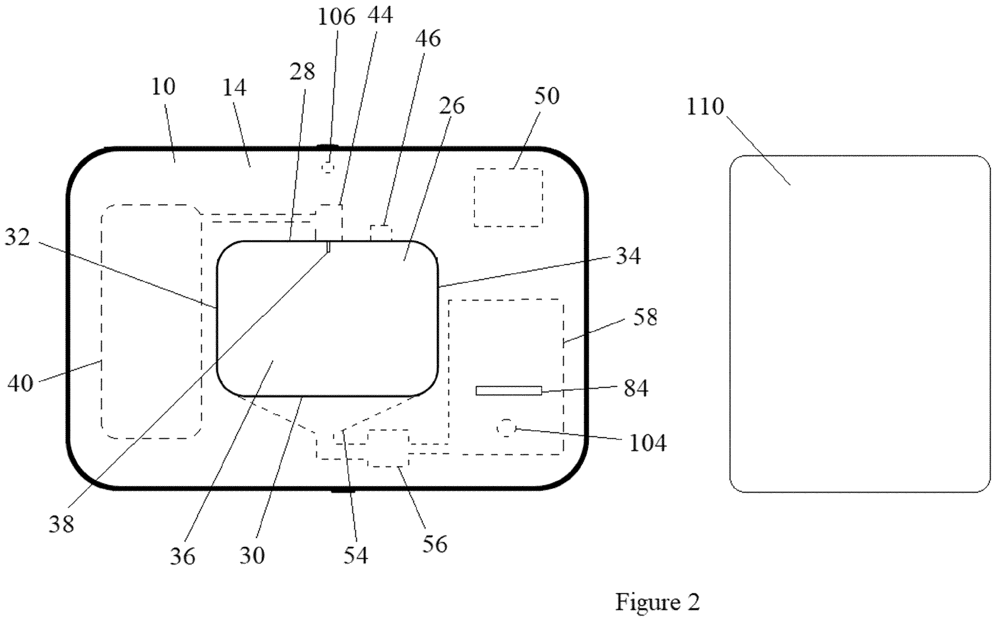

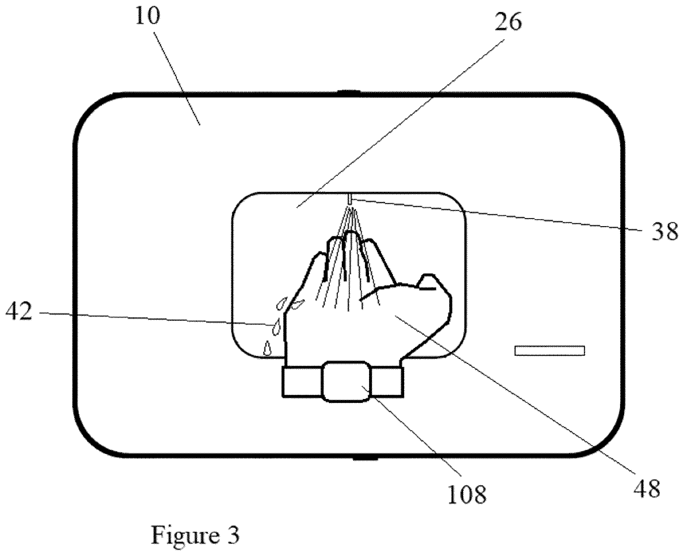

The chamber 26 is defined by an upper wall 28, a lower wall 30, a right lateral wall 32, a left lateral wall 34, and a rear wall 36. As can be seen in Figure 2, a fluid outlet 38 extends downwardly from the upper wall 28. As shown schematically in Figure 2, the housing 12 contains a fluid reservoir 40 containing a hand cleaning fluid 42, and a pump mechanism 44 that dispenses the fluid 42 from the fluid outlet 38 when the pump mechanism 44 is activated. Any suitable fluid reservoir 40 and pump mechanism 44 could be used. Preferably, the dispenser 10 includes at least one sensor 46 that is able to detect when a user's hand 48 is placed inside the chamber 26. The pump mechanism 44 is preferably configured to activate when the user's hand 48 is detected within the chamber 26, which causes the fluid 42 to be dispensed from the fluid outlet 38 onto the user's hand 48, as shown in Figure 3.

As can be seen in Figure 1, the lower wall 30 of the chamber 26 has a plurality of apertures 52. The apertures 52 allow fluid 42 that falls onto the lower wall 30 to pass through the lower wall 30 and into a fluid collector 54 that is positioned below the lower wall 30. The fluid collector 54 is shown schematically in Figure 2 by dotted lines. The fluid collector 54 delivers the fluid 42 to a fluid analyzer 56, also shown schematically in Figure 2 by dotted lines.

The fluid analyzer 56 is configured to analyze the fluid 42 to detect contaminants present therein. In preferred embodiments, the fluid analyzer 56 is configured to detect biological particles, such as viruses, bacteria, spores, yeast, and/or fungi. The fluid analyzer 56 may have any structure suitable for analyzing the fluid 42 in order to detect materials, components, particles, and/or contaminants present therein. The fluid analyzer 56 may, for example, have one or more of the structures disclosed in United States Patent No. 9,437,103 to Ophardt, issued September 6, 2016 and United States Patent Application Publication No. 2022/0091011 to Steltenkamp et al., published March 24, 2022, which are incorporated herein by reference.

The fluid dispenser 10 also includes a sample preparation mechanism 58, which is shown schematically in Figure 2. The sample preparation mechanism 58 is configured to receive at least some of the fluid 42 collected by the fluid collector 54, and to use the fluid 42 to generate a sample 60 for external analysis.

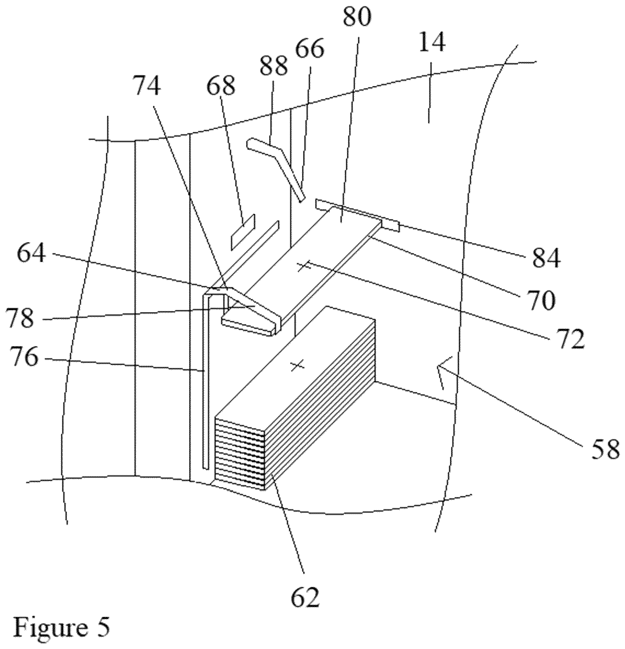

A more detailed internal view of the sample preparation mechanism 58 is shown in Figures 5 to 9. The sample preparation mechanism 58 includes a substrate storage area 62, a substrate manipulation device 64, a specimen delivery mechanism 66, and a drying device 68. The substrate storage area 62 is positioned behind the front wall 14 of the housing 12, and holds a stack of glass slides 70. Preferably, the glass slides 70 each include a specimen marker 72, which for example may be in the form of a cross or other marking on the slide 70.

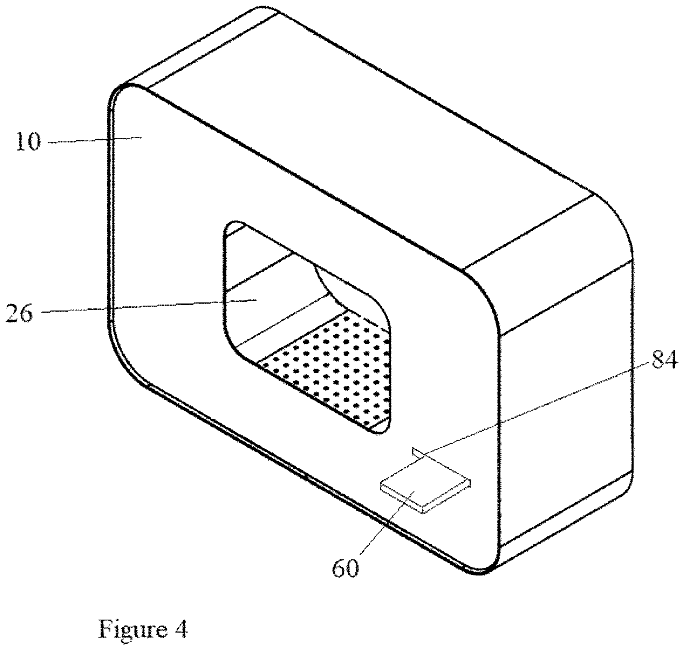

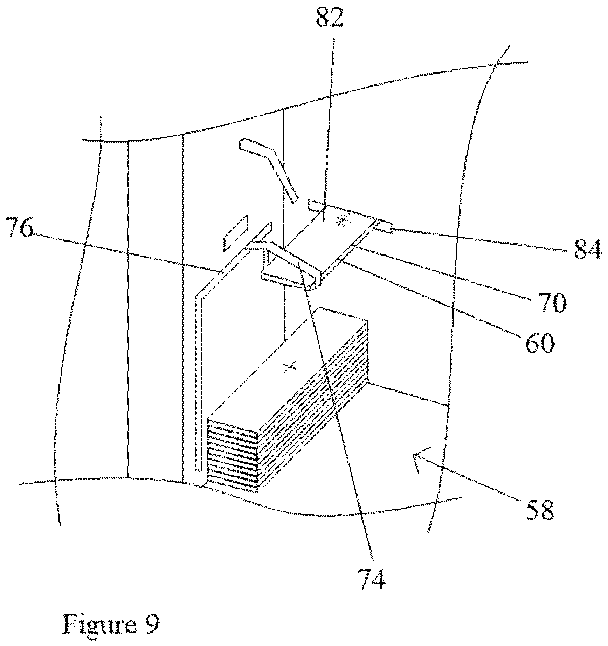

The substrate manipulation device 64 includes an arm 74 that is moveable relative to the housing 12 along a transport channel 76. The arm 74 has a slide gripping portion 78 that is configured to grasp the glass slides 70 individually. The arm 74 is configured to move the glass slides 70 from the substrate storage area 62 to a sample preparation position 80. In the embodiment shown in Figure 5, the sample preparation position 80 is located above the substrate storage area 62. The arm 74 moves a glass slide 70 from the substrate storage area 62 to the sample preparation position 80 by grasping the uppermost slide 70 in the stack, and then travelling upwardly along the transport channel 76. The arm 74 is also able to move the slide 70 from the sample preparation position 80 shown in Figure 5 to the sample discharge position 82 shown in Figures 4 and 9, by travelling forwardly along the transport channel 76. When in the sample discharge position 82 shown in Figures 4 and 9, the slide 70 extends outwardly from the housing 12 through a discharge slot 84 that extends through the front wall 14 of the housing 12.

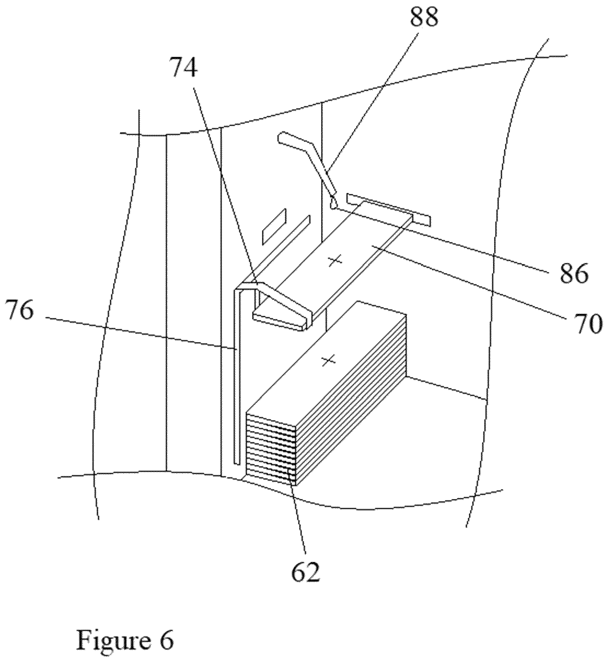

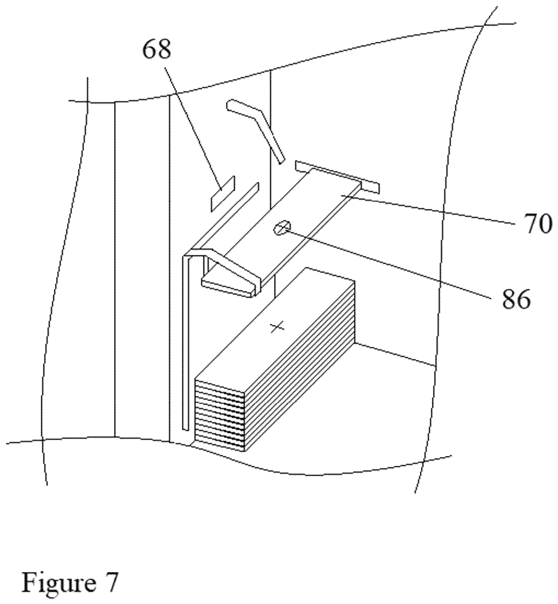

The specimen delivery mechanism 66 is configured to deliver a sample fluid 86 onto a glass slide 70 that is positioned in the sample preparation position 80, as shown in Figures 6 and 7. In the embodiment shown, the sample delivery mechanism 66 comprises an outlet tube 88 that is positioned above the sample preparation position 80, though any suitable mechanism for delivering the sample fluid 86 to the slide 70 could be used.

In some embodiments of the invention, the sample fluid 86 is simply the hand cleaning fluid 42 collected by the fluid collector 54. In other embodiments of the invention, the hand cleaning fluid 42 collected by the fluid collector 54 is processed in order to generate the sample fluid 86. For example, the hand cleaning fluid 42 may be concentrated, diluted, filtered, or washed in order to generate the sample fluid 86. Various components may also be added or removed from the fluid 42, and/or chemical properties such as pH or osmotic pressure may be adjusted. Various dyes and/or biochemical labels or markers may also be added to the fluid 42. In some embodiments of the invention, the particles that are present in the fluid 42 may be sorted and/or concentrated, for example using a microfluidic particle sorter, and the sample fluid 86 may be generated from one or more of the sorted and/or concentrated streams. In some embodiments of the invention, the hand cleaning fluid 42 may be completed replaced with a new sample fluid 86, with the biological particles that were present in the hand cleaning fluid 42 being removed and re-suspended in the sample fluid 86.

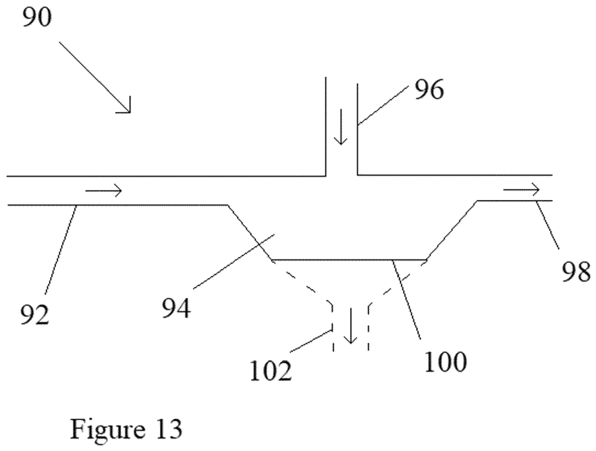

An example of an internal fluid processing channel 90 that may be used to process the hand cleaning fluid 42 in order to generate the sample fluid 86 is shown in Figure 13. The fluid processing channel 90 includes a fluid input passage 92, a mixing chamber 94, an additive input passage 96, and an output passage 98. The fluid input passage 92 receives the hand cleaning fluid 42 collected by the fluid collector 54 and delivers the fluid 42 to the mixing chamber 94. The hand cleaning fluid 42 may be received directly from the fluid collector 54 or from the fluid analyzer 56.

Optionally, one or more additives may be delivered to the mixing chamber 94 via the additive input passage 96. The additives may include, for example, one or more dyes, one or more biochemical markers or labels, a buffer solution, and/or chemicals to raise or lower the pH of the fluid 42.

Optionally, the mixing chamber 94 includes one or more semipermeable barriers 100 that allow some of the components of the fluid 42 to drain out of the mixing chamber 94 and into a disposal chute 102. For example, the semipermeable barrier 100 may allow alcohol to drain out of the mixing chamber 94, thereby lowering the alcohol concentration of the fluid 42. In some embodiments of the invention, the hand cleaning fluid 42 may be entirely discarded via the disposal chute 102, with only the biological particles that were suspended in the fluid 42 being left behind on the semipermeable barrier 100. The particles can then be suspended in a sample fluid 86 received via the additive input passage 96. The sample fluid 86 is then transported to the specimen delivery mechanism 66 via the output passage 98.

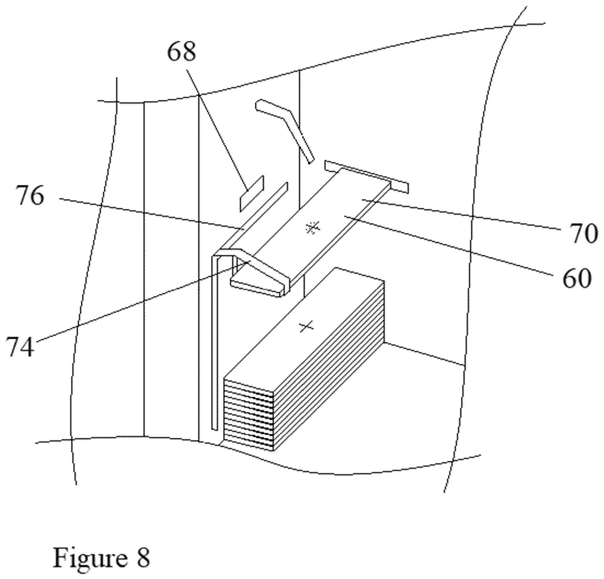

Referring to Figures 7 and 8, once the sample fluid 86 is deposited onto the glass slide 70, the drying device 68 can be used to dry the fluid 86. This leaves behind any biological particles that were present in the fluid 86 stuck to the surface of the slide 70, as well as certain additives that may be present such as dyes or biological markers. The drying device 68 may for example apply heat and/or ventilation to dry the fluid 86. Alternatively, in some embodiments of the invention the drying device 68 may be omitted, and for example a cover slip could be placed over the sample fluid 86 rather than drying the fluid 86. The cover slip could optionally be glued in place.

Preferably, the dispenser 10 includes at least one electronic controller assembly 50, shown schematically in Figure 2. The controller assembly 50 preferably includes one or more electronic components, such as a processor, a memory, a communication device, and a power source. The controller 50 is preferably programmed to control one or more of the pump mechanism 44, the fluid analyzer 56, and the sample preparation mechanism 58.

A method of using the hand cleaning fluid dispenser 10 will now be described with reference to Figures 1 to 9. The fluid dispenser 10 is preferably installed at a location where individuals are required or encouraged to clean their hands, such as in a washroom, the entrance to a hospital or long term care facility, and/or a patient's room in a hospital. The dispenser 10 may, for example, be mounted to a vertical wall or post.

In order to activate the dispenser 10, a user places his or her hand 48 into the chamber 26, as shown in Figure 3. This triggers activation of the pump mechanism 44, which delivers the hand cleaning fluid 42 from the fluid outlet 38 onto the user's hand 48. Any suitable hand cleaning fluid 42 may be used, such as a hand sanitizer or hand soap. Optionally the fluid 42 may contain alcohol.

The fluid dispenser 10 is preferably configured to produce an overspray of hand cleaning fluid 42, such that the fluid 42 drips off of the user's hand 48 and into the fluid collector 54. This may be achieved, for example, by dispensing a large volume of the fluid 42 onto the user's hand 48, with the result that excess fluid 42 inevitably runs off of the sides of the user's hand 48 and into the fluid collector 54. Other adaptations that may be used to encourage overspray are disclosed in United States Patent Application Publication No. 2024/0099704 to Ophardt et al., published 28 March 2024, which is incorporated herein by reference.

The collected fluid 42 is then transported to the fluid analyzer 56, which preferably performs a preliminary analysis of the fluid 42. For example, the fluid analyzer 56 may be configured to detect, count, classify, and/or identify biological particles from the user's hand 48 that are suspended in the fluid 42. The fluid analyzer 56 may, for example, incorporate one or more features disclosed in one or more of: United States Patent Application Publication No. 2022/0091011 to Steltenkamp et al., published 24 March 2022; United States Patent Application Publication No. 2024/0099704 to Ophardt et al., published 28 March 2024; and co-pending United States Patent Application Serial No. 18/669,881 to Ophardt et al., filed 21 May 2024, which are incorporated herein by reference.

The preliminary analysis provided by the fluid analyzer 56 preferably informs the operation of the sample preparation mechanism 58. For example, if the preliminary analysis reveals the possible presence of an infection, the sample preparation mechanism 58 may be configured to prepare a sample 60 for external analysis. Alternatively, in some embodiments of the invention the fluid analyzer 56 may be omitted entirely, and other criteria may be used to govern the preparation of samples 60. For example, the fluid dispenser 10 may optionally include a button 104, shown in dotted lines in Figure 2, which can be pressed by a user in order to trigger the preparation of a sample 60. Alternatively, the dispenser 10 may be configured to prepare samples 60 from a random selection of users, or in accordance with any other suitable sampling protocol.

In some preferred embodiments of the invention, the dispenser 10 is configured to identify at least some of the users of the dispenser 10. For example, the dispenser 10 may be able to identify some or all of the employees of the facility where the dispenser 10 is located. The dispenser 10 may, for example, include a camera 106 (shown in dotted lines in Figure 2) which captures images of the users. The images may then be analyzed using facial recognition software to identify the users. Alternatively, the dispenser 10 may be able to communicate with an identification device carried by the users in order to identify the users. Any suitable identification device may be used, such as an identification card or badge, a key fob, a smart phone, or a smart watch 108 as shown in Figure 3. The dispenser 10 may be configured to prepare samples 60 from specific individuals, such as the clinical staff of a hospital, and to refrain from preparing samples 60 from other individuals, such as hospital visitors.

In the embodiment shown in Figures 5 to 9, a sample 60 is prepared by depositing a drop of sample fluid 86 onto a glass slide 70 and then drying the sample fluid 86 using the drying device 68, as described above. The sample 60 is then moved to the sample discharge position 82 as shown in Figures 4 and 9. When in the sample discharge position 82, the sample 60 can be manually removed from the dispenser 10 by grabbing the slide 70 and pulling it forwardly out through the discharge slot 84. The sample 70 can then be brought to an external analysis device 110, shown schematically in Figure 2.

The external analysis device 110 may include any machine or device that can be used for analysis of the sample 60, including an optical microscope, an electron microscope, a scanning electron microscope, a Raman imaging spectroscope, a polymerase chain reaction thermocycler, a centrifuge, a mass spectrometer, a genetic sequencer, an incubator, a colony counter, and/or a flow cytometer.

Preferably, the sample 60 is prepared specifically for the type of external analysis that is to be performed. For example, in the embodiment shown in Figures 1 to 9, the sample 60 is prepared for analysis using an optical microscope. The placement of the sample fluid 86 on the specimen marker 72 preferably makes it easy for a laboratory technician to locate the specimen on the glass slide 70, by aligning the specimen marker 72 with the field of view of the microscope. The technician can then perform an analysis of the sample 60, such as identifying the types of biological particles that are present. This may be aided by, for example, dyes or other biological markers or labels that are preferably deposited onto the sample 60 by the dispenser 10 itself.

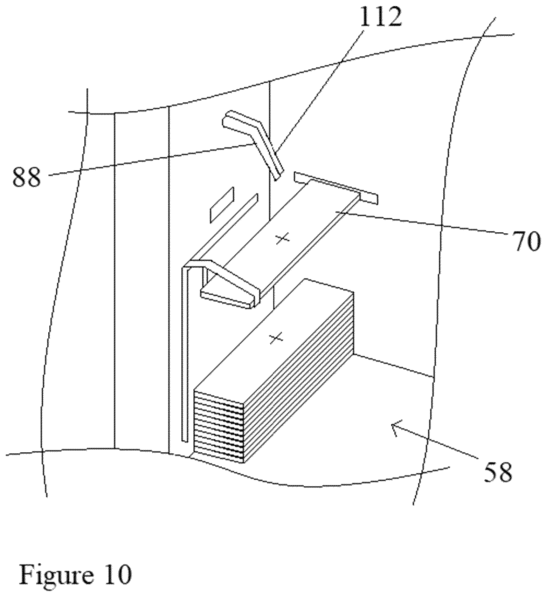

An alternative construction of the sample preparation mechanism 58 is shown in Figure 10. In this construction, there is a secondary outlet tube 112 positioned adjacent to the sample outlet tube 88. The secondary outlet tube 112 may be used, for example, to deposit dyes or other biological markers or labels onto the sample 60. Alternatively, other fluids such as fixing agents, preservatives, contrast agents, mounting media, immersion oils, anti-fade agents, and/or clearing agents may be applied using the secondary outlet tube 112. Additional outlet tubes could also be incorporated into the sample preparation mechanism 58 for depositing additional additives.

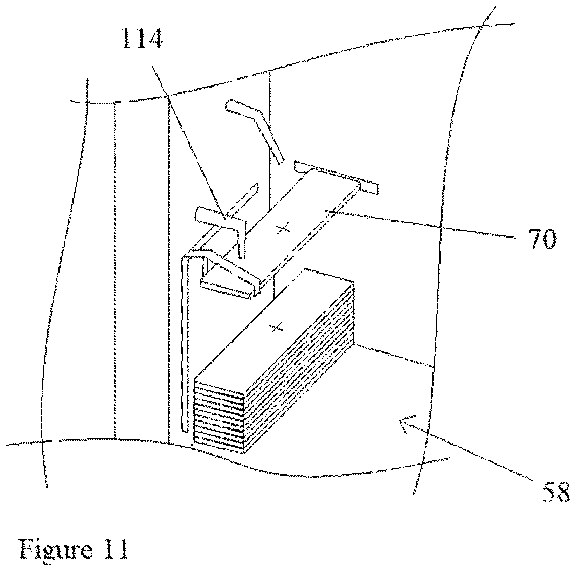

A further alternative construction of the sample preparation mechanism 58 is shown in Figure 11. In this construction, the sample preparation mechanism 58 includes a laser engraving device 114. The laser engraving device 114 may be used to add a label to the sample 60. The label may include any useful or potentially useful information, such as the identity of the user from which the sample 60 was collected, contact information for the user, the identification number of the dispenser 10, the time and location where the sample 60 was collected, and/or a summary of any preliminary analysis performed by the fluid analyzer 56. This information can then be used by the laboratory technician performing the external analysis. For example, if the external analysis reveals that the user has a concerning infection, the laboratory technician can use the label to identify and contact the user so that appropriate actions may be taken, such as isolating and seeking medical treatment.

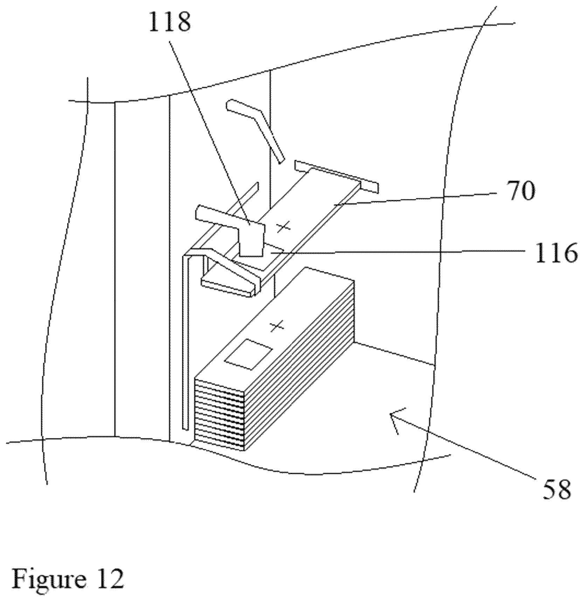

Another alternative construction of the sample preparation mechanism 58 is shown in Figure 12. In this construction, the glass slides 70 are provided with pre-applied labels 116. The labels 116 may, for example, include a barcode or other unique number or code that can be used to distinguish the slides 60 from each other. The sample preparation mechanism 58 also includes a label reader 118, which may for example be in the form of a barcode reader or the like. The label reader 118 is preferably configured to read the label 116 of the glass slide 70 that is in the sample preparation position 80. The label information is then transmitted to the controller assembly 50, and is linked with information about the sample 60, such as the identity of the user from which the sample 60 was collected, contact information for the user, the identification number of the dispenser 10, the time and location where the sample 60 was collected, and/or a summary of any preliminary analysis performed by the fluid analyzer 56. This information is then stored in a database, which may for example be stored on an external server. The information can then be retrieved by a laboratory technician, for example by scanning the label 116 of a sample 60 that is being analyzed.

The fluid dispenser 10 could be configured to generate any suitable form of sample 60, and is not limited a specimen deposited on a glass slide 70 as shown in Figures 1 to 9. For example, the fluid dispenser 10 could be configured to generate a sample 60 in the form of a silicon wafer or stub prepared for scanning electron microscopy. The fluid dispenser 10 may, for example, deposit a specimen derived from the hand cleaning fluid 42 onto a silicon wafer, dry the specimen using for example critical point drying or freeze drying, and coat the specimen with a conductive material such as gold.

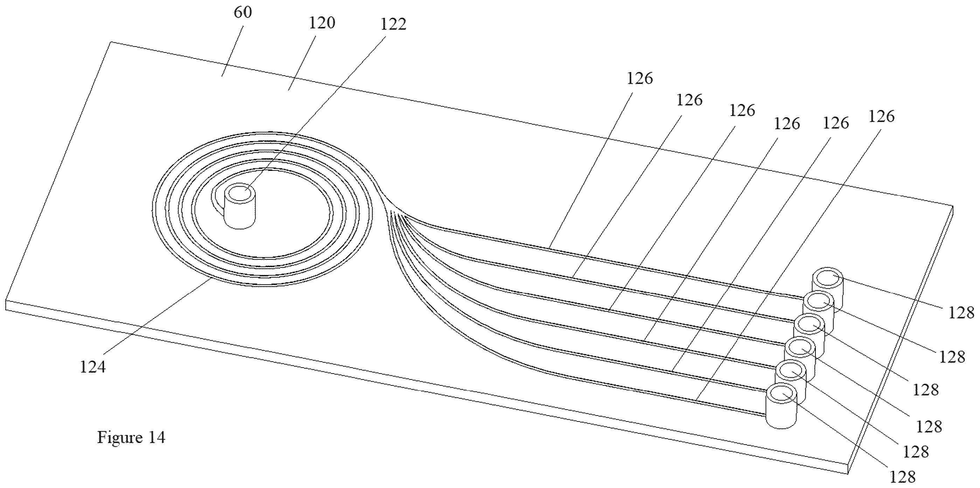

In other embodiments of the invention, the dispenser 10 could prepare a sample 60 in the form of a microfluidic chip 120, as shown for example in Figure 14. In the embodiment shown in Figure 14, the chip 120 has a fluid inlet port 122, a particle sorting channel 124, and six output channels 126 connected to six output ports 128. The fluid inlet port 122 receives the sample fluid 86, and delivers the sample fluid 86 to the particle sorting channel 124. As the fluid 86 travels through the particle sorting channel 124, the biological particles that are present in the fluid 86 are separated into separate streams by size and/or shape, as is described in more detail in United States Patent Application Publication No. 2022/0091011 to Steltenkamp et al., published 24 March 2022, which is incorporated herein by reference. The separate streams of particles are each delivered to a separate one of the output channels 126, and can be removed from the chip 120 for external analysis via the output ports 128.

The microfluidic chip 120 is thus configured to provide a degree of pre-processing of the sample fluid 86, in this case by separating the biological particles according to size and/or shape. In other embodiments of the invention, the chip 120 could be configured to pre-process the fluid 86 in any desired manner, such as separation of particles by magnetic and/or electrical characteristics, or by adding different additives to each of the output channels 126 to prepare the fluid 86 within each output channel 126 for a different type of external analysis.

Optionally, the microfluidic chip 120 may be employed in the preliminary analysis performed by the fluid analyzer 56. For example, the fluid analyzer 56 may include one or more optical microscopes that capture images of the particles travelling through each of the output channels 126. The images may then be analyzed to, for example, determine if there is an unusual or unexpected concentration of particles of a given shape and/or size in the fluid 86. This information may then be included in a report that is automatically generated and transmitted to the laboratory technician. The report may, for example, indicate an unusually large number of particles detected in one of the outlet channels 126, and recommend that a particular external analysis (such as optical microscopy or scanning electron microscopy) be performed on the fluid 86 contained in that particular outlet channel 126.

Each sample 60, whether in the form of a glass slide 70, microfluidic chip 120, or other, may optionally include: one specimen collected from one user, prepared for one type of external analysis; multiple specimens collected from one user, prepared for multiple different types of external analysis; multiple specimens collected from multiple users, prepared for one type of external analysis; multiple specimens collected from multiple users, prepared for multiple different types of external analysis; and/or any other combination of number of specimens, number of users, and number of external analyses.

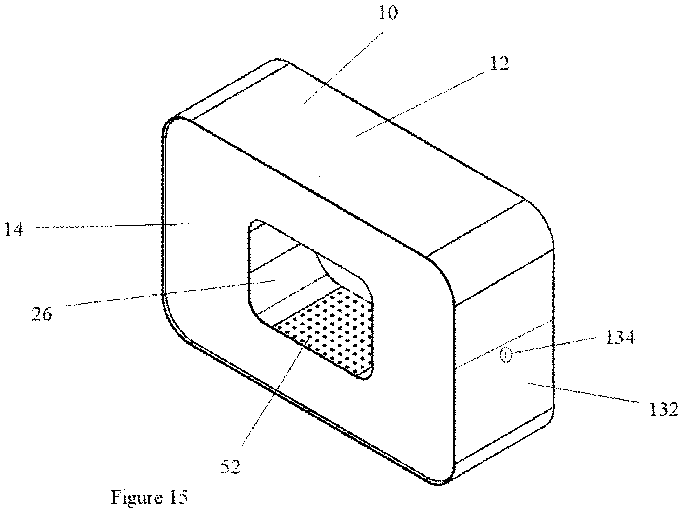

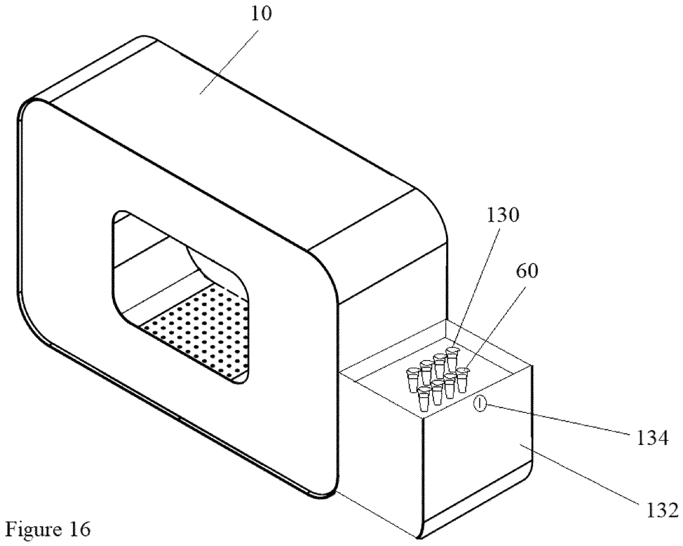

A fluid dispenser 10 in accordance with a second embodiment of the invention is shown in Figures 15 to 17. Like numerals are used to denote like components. The fluid dispenser 10 shown in Figures 15 to 17 is configured to prepare samples 60 in the form of sample vials 130. Each sample vial 130 contains a sample fluid 86 derived from the hand cleaning fluid 42, and which preferably has been processed to prepare for one or more forms of external analysis. For example, the hand cleaning fluid 42 may have been replaced with a buffer or preservative solution. Alternatively or in addition, the sample 60 may be prepared for PCR analysis, for example by homogenizing the sample 60 with blending, extracting DNA with a lysis buffer and proteinase, and incubating the sample 60 at a suitable temperature. Alternatively, the sample 60 may be prepared for biological culturing, for example by replacing the hand cleaning fluid 42 with a suitable culture medium, and storing the sample 60 at a suitable temperature. Any suitable sample fluid 86 could be used, and the invention is not limited to sample fluids 86 containing any particular component or components.

The fluid dispenser 10 shown in Figures 15 to 17 has a storage container 132 for storing one or more samples 60 after the samples 60 have been prepared. Preferably, the storage container 132 has a locking mechanism 134 for preventing unauthorized access to the samples 60. The locking mechanism 134 may, for example, be a lock that can be opened with a key. The key can be given to a laboratory technician or other worker authorized to handle the samples 60.

Any suitable means of delivering the samples 60 to the external analysis device 110 may be used. For example, the user of the dispenser 10 may retrieve his or her sample 60, and deliver the sample 60 to the laboratory where the external analysis device 110 is located. Alternatively, the sample 60 could be collected by a laboratory technician. Preferably, the dispenser 10 is configured to send an alert to the laboratory technician when a sample 60 is ready to be picked up, such as by sending a text message or e-mail alert.

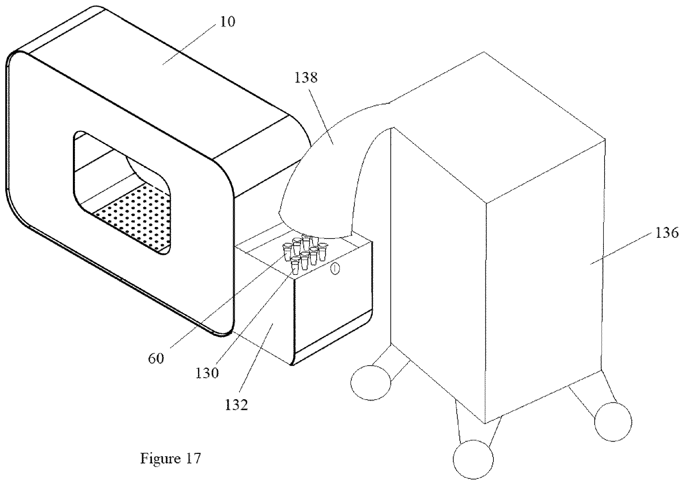

In the embodiment shown in Figure 17, the fluid dispenser 10 is part of a system that includes at least one delivery robot 136. The robot 136 is configured to collect the samples 60 from the storage container 132, and deliver the samples 60 to the laboratory where the external analysis device 110 is located. In the example shown, the robot 136 has a vacuum tube 138 that is used to retrieve the samples 60 from the dispenser 10, though any suitable mechanism for retrieving the samples 60 could be used. In some embodiments of the invention, the robot 136 may be enabled to deliver the samples 60 directly to the external analysis device 110, and/or to initiate the external analysis.

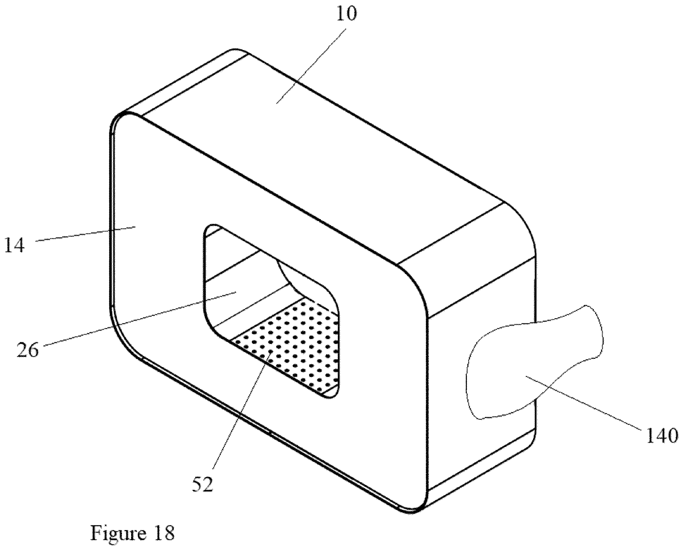

A further embodiment of the fluid dispenser 10 is shown in Figure 18. In this embodiment of the invention, the fluid dispenser 10 has a pneumatic outlet 140 that is connected to a pneumatic transport system (not shown) installed in the facility where the fluid dispenser 10 is located. The pneumatic transport system uses compressed air or gas to carry objects through a network of tubes within the facility. The fluid dispenser 10 is configured to deliver its samples 60 to the pneumatic transport system via the pneumatic outlet 140. The pneumatic transport system then carries the samples 60 to the laboratory where the external analysis device 110 is located.

It will be understood that, although various features of the invention have been described with respect to one or another of the embodiments of the invention, the various features and embodiments of the invention may be combined or used in conjunction with other features and embodiments of the invention as described and illustrated herein.

The invention is not limited to the particular structures of the preferred embodiments that have been shown in the drawings. Rather, any functionally equivalent structures could be used.

For example, the fluid dispenser 10 need not have the particular shape and structure shown in the drawings. Rather, any suitable structure that allows fluid 42 to be dispensed onto a user's hand 48, and overspray to be collected for the preparation of a sample 60, could be used. The fluid dispenser 10 may, for example, incorporate any suitable structure or feature disclosed in any one or more of: United States Patent Application Publication No. 2022/0091011 to Steltenkamp et al., published 24 March 2022; United States Patent Application Publication No. 2024/0099704 to Ophardt et al., published 28 March 2024; co-pending United States Patent Application Serial No. 18/669,881 to Ophardt et al., filed 21 May 2024; United States Patent No. 8,245,877 to Ophardt, issued August 21, 2012; United States Patent No. 8,113,388 to Ophardt et al., issued February 14, 2012; United States Patent No. 8,091,739 to Ophardt et al., issued January 10, 2012; United States Patent No. 7,748,573 to Anhuf et al., issued July 6, 2010; U.S. Patent No. 7,984,825 to Ophardt et al., issued July 26, 2011; U.S. Patent No. 8,684,236 to Ophardt, issued April 1, 2014; U.S. Patent No. 5,373,970 to Ophardt, issued December 20, 1994; U.S. Patent No. 5,836,482 to Ophardt et al., issued November 17, 1998; U.S. Patent No. 10,893,780 to Ophardt et al., issued 19 January 2021; and U.S. Patent No. 9,682,390 to Ophardt et al., issued June 20, 2017, which are incorporated herein by reference.

The fluid analyzer 56 may be configured to use any suitable method of analyzing the fluid 42, including, for example, transversal or longitudinal waveforms, electrical methods (e.g. impedance), and thermal methods such as heating/cooling. The fluid analyzer 56 may use one or more of: optical waveforms, acoustic waveforms, electrical waveforms, and techniques such as bright-field, dark-field microscopy, scattering of light, optical traps or ultrasound, for example.

The sample preparation mechanism 58 is also not limited to the particular examples shown in the drawings. Rather, any suitable mechanism for preparing a sample 60 for external analysis could be used.

In some embodiments of the invention, the sample preparation mechanism 58 is only able to prepare one type of sample 60. In other embodiments of the invention, the sample preparation mechanism 58 may be able to prepare more than one type of sample 60. In embodiments in which the sample preparation mechanism 58 can prepare more than one type of sample 60, the fluid dispenser 10 may optionally be configured to determine which type of sample 60 to prepare based on different criteria. For example, the sample preparation mechanism 58 may be directed to prepare a first type of sample 60 from certain individuals (e.g. clinical staff at a hospital), and a second type of sample 60 from other individuals (e.g. patients in a hospital). The fluid dispenser 10 may also rely on the preliminary analysis performed by the fluid analyzer 56 in order to determine which type of sample 60 to prepare. For example, if the preliminary analysis shows an elevated concentration of bacteria, the sample preparation mechanism 58 may be directed to prepare a sample 60 for optical microscopy and/or bacterial culturing. Alternatively, if the preliminary analysis shows an elevated concentration of viral particles, the sample preparation mechanism 58 may prepare a sample 60 for scanning electron microscopy, or Enzyme-Linked Immunosorbent Assay (ELISA).

In some embodiments of the invention, the fluid analyzer 56 may be configured to categorize particles detected in the fluid 42 into known and unknown classes. An example of a method for categorizing particles into known and unknown classes is described in co-pending United States Patent Application Serial No. 18/669,881 to Ophardt et al., filed 21 May 2024, which is incorporated herein by reference. If unknown particles are detected, or if an elevated concentration of unknown particles are detected, the dispenser 10 is preferably configured to direct the sample preparation mechanism 58 to prepare at least one sample 60 for external analysis, and more preferably to prepare multiple samples 60 for a variety of different types of external analyses, so that the unknown particle can be quickly identified and/or characterized.

In some embodiments of the invention, the fluid dispenser 10 can receive instructions from an external source as to the types of samples 60 to prepare, and/or the sampling protocol to be used. For example, a laboratory technician may be able to send instructions to the dispenser 10 electronically, e.g. via Wi-Fi, Bluetooth, or Near-Field Communication, to direct the dispenser 10 as to the type(s) of samples 60 to prepare.

Preferably, the dispenser 10 is able to receive feedback from the external analyses that are performed on the samples 60. For example, if the fluid analyzer 56 detects a possible infection, but the external analysis reveals that there is no infection, this result is preferably communicated back to the dispenser 10 as training data, in order to improve the ability of the dispenser 10 to detect infections. As another example, if the dispenser 10 is unable to identify a particular biological particle, and the identity of the particle is later determined via the external analysis, this information can be transmitted back to the dispenser 10 as training data, to improve the ability of the dispenser 10 to identify the particle.

The external analyses of the samples 60 may be used, for example, to determine the degree of contamination, the ratio of dead and alive biological particles, the infection probability of the user, the composition of the biological species, the make-up of the skin microbiota of the user, and/or the hazardous potential of the pathogens detected and of the user moving with respect to the environment.

Any suitable technique for preparing the sample fluid 86 could be used, including one or more of: liquid exchange, increasing or decreasing alcohol concentration, adding fluidic/solid components, such as micro particles of dye, adjusting the pH, viscosity or any other chemical, physical or biological properties. Optionally, the fluid 86 can be divided into several portion using a fluidic separator, for example using microfluidic droplet generators. Each portion of the fluid 86 can then be used for a different external analysis.

In some embodiments of the invention, the sample preparation process may include one or more of the following steps: placing the sample fluid 86 on a sample holder, e.g. a glass slide, metal sheet, or any other compartment; drying the fluid 86, for example by heat or evaporation, optionally aided by ventilation of a gas; sealing the specimen by any suitable techniques, such as putting a lid or cover on the specimen; coating the specimen with a metal or similar to increase conductivity, light-transmission or reflectivity, for example by means of thermal evaporation, sputtering, etc. It is also possible that wet specimens are transferred by means of a stamp or by means of embossing on to a sample holder (e.g. micro slide, glass substrate, metal sheet, etc.). Then the specimen dries out - this can be supported by heating or vacuum techniques. After all physical, chemical and biological properties are adjusted, the specimen may, for example, be packed or sealed in a microfluidic chip, fluidic container or similar. Alternatively, the liquid specimen could also be cast into, for example, PDMS (Polydimethylsiloxane), wax or epoxy resin. It is also possible that the specimen is packed by vacuum-techniques.

One example of a method of preparing a sample 60 is as follows: first a droplet of the sample fluid 86 is dried on a glass slide 70. The drying process can optionally be supported by heat. After the fluid 86 is half dried, a fixation step can be performed. For example, glutaraldehyde or paraformaldehyde can be sprayed onto the specimen and dried. In this case the shape of the particles is conserved, but the active biological properties are suppressed. By using a resin, the specimen can be sealed.

In preferred embodiments, the samples 60 are labelled so as to include useful information, such as the identity of the user from which the sample 60 was taken. Any suitable method of labelling the samples 60 could be used, including printing the labels using ink, or engraving the labels using a laser. Any suitable method of identifying the user could be used, including facial recognition, fingerprint detection, iris scanning, and/or manual input of identifying information. Preferably, information related to each sample 60 is stored on an external database, which is accessible by laboratory staff and/or facility managers. Alerts are preferably automatically generated, for example to alert the user when a possible infection is detected, to alert laboratory staff when a possible infection is detected, and/or to alert laboratory staff when a sample 60 is ready for external analysis.

In some embodiments of the invention, the sample 60 that is prepared may be in the form of a microfluidic chip 120 having multiple different output ports 128. The fluid 86 in each output port 128 may be processed by the chip 120 so as to be prepared for a different type of external analysis. For example, one output port 128 may contain fluid 86 that has a dye added, to be used for optical microscopy, and another output port 128 may have a broth media for cultivation purposes. Although the microfluidic chip 120 shown in Figure 14 includes a particle sorting channel 124, this is not necessary. In other embodiments of the invention, the chip 120 may prepare the sample fluid 86 for multiple different external analyses, without separating the particles into different streams. Preferably, all of the ports 122, 128 of the chip 120 are sealed when the chip 120 is ready to be removed from the dispenser 10, to avoid contamination of the sample 60.

In some embodiments of the invention, the sample 60 may be positioned within the dispenser 10 on a stage or sample holder that is configured to provide controlled heating, stirring, and/or evaporation. In some embodiments, the stage may also cool the sample 60, for example to provide a controlled evaporation process.

In the embodiment of the invention shown in Figure 2, the sensor 46 is described as detecting the presence of a user's hand 48 in the chamber 26. Any type of sensor 46 that is capable of detecting a hand 48 could be used, including for example an infrared sensor, a proximity sensor, or a time of flight sensor. Other types of sensors 46 could also be incorporated into the dispenser 10, such as a touchless thermometer to detect the user's body temperature, and/or a microphone to detect sounds indicative of a possible infection, such as coughing and/or sneezing.

The word "specimen" as used herein refers to any product derived from the hand cleaning fluid 42 collected by the fluid collector 56 that forms the part of the sample 60 to be analyzed by the external analysis device 110. The specimen may, for example, be in the form of a solution, a liquid, a fluid, a mixture, a powder, and/or a solid. The specimen may contain intact biological particles and/or biological particles that have been modified, killed, dried, digested, and/or denatured. The word "substrate" as used herein refers to any product or material that forms the part of the sample 60 that carries and/or contains the specimen. The substrate may, for example, be in the form of a glass slide 70, a silicon wafer, a metal sheet, a microfluidic chip 120, and/or a sample container or sample vial 130.

The term "fluid" as used herein is intended to refer broadly to any flowable substance, including liquids, gels, foams, and emulsions. The hand cleaning fluid 42 that is dispensed by the hand cleaning fluid dispenser 10 may include, for example, soap, sanitizer, and/or disinfectant.

Although this disclosure has described and illustrated certain preferred embodiments of the invention, it is to be understood that the invention is not restricted to these particular embodiments. Rather, the invention includes all embodiments which are functional, optical, electrical, or mechanical equivalents of the specific embodiments and features that have been described and illustrated herein.

## Claims

1. A fluid dispenser (10) comprising:
a fluid outlet (38) for dispensing a fluid (42) onto a user's hand (48);
a fluid collector (54) for collecting at least some of the fluid (42) after the fluid (42) has contacted the user's hand (48); and
a sample preparation mechanism (58) for generating a sample (60) from the fluid (42) collected by the fluid collector (54), the sample (60) being removable from the fluid dispenser (10) for external analysis.

2. The fluid dispenser (10) according to claim 1, wherein the sample preparation mechanism (58) processes the fluid (42) collected by the fluid collector (54) to prepare the sample (60) for the external analysis.

3. The fluid dispenser (10) according to claim 1 or 2, wherein the fluid (42) dispensed from the fluid outlet (38) is a hand cleaning fluid (42).

4. The fluid dispenser (10) according to any one of claims 1 to 3, wherein the sample preparation mechanism (58) is configured to extract particles from the fluid (42) and suspend the particles in a sample fluid (86).

5. The fluid dispenser (10) according to claim 4, wherein the sample fluid (86) comprises at least one of: a buffer fluid, a cultivation broth, and a preservation fluid.

6. The fluid dispenser (10) according to any one of claims 1 to 5, wherein the sample (60) comprises a substrate and a specimen;
wherein the specimen comprises at least some of the fluid (42) collected by the fluid collector (54) or a processed product generated from the fluid (42) collected by the fluid collector (54); and
wherein the sample preparation mechanism (58) is configured to: deposit the specimen onto the substrate and/or deliver the specimen into the substrate.

7. The fluid dispenser (10) according to claim 6, wherein the substrate comprises at least one of: a microscope slide (70), a microfluidic chip (120), a sample vial (130), a silicon wafer, and a scanning electron microscopy mount or stub.

8. The fluid dispenser (10) according to claim 6 or claim 7, wherein the sample preparation mechanism (58) is configured to at least one of:
dry the specimen deposited onto the substrate;
apply a dye to at least one of: the specimen and the substrate;
apply a sealing fluid to the specimen deposited onto the substrate;
coat the specimen in gold;
perform at least one fixation step on the sample (60) and/or the specimen;
apply a fixative fluid to the sample (60) and/or the specimen;
heat the sample (60) and/or the specimen;
cool the sample (60) and/or the specimen;
adjust a pH of the sample (60) and/or the specimen;
prepare the sample (60) for analysis in a laboratory;
prepare the sample (60) for microscopy;
prepare the sample (60) for scanning electron microscopy;
prepare the sample (60) for Raman spectroscopy;
prepare the sample (60) for a polymerase chain reaction (PCR);
prepare the sample (60) for microbe culturing;
coat the specimen in metal;
coat the specimen in a polymer;
prepare the sample (60) for hyperspectral imaging;
prepare the sample (60) for fluorescence imaging;
separate particles present in the fluid (42) by size;
separate particles present in the fluid (42) based on one or more particle characteristics;
separate particles present in the fluid (42) based on electrical properties of the particles;
separate particles present in the fluid (42) using a microfluidic particle sorter;
dilute the fluid (42);
adjust a concentration of the fluid (42);
extract a component from the fluid (42);
purify the specimen;
perform a liquid exchange;
adjust a viscosity of the fluid (42); and
adjust one or more chemical, physical, and/or biological properties of the sample (60).

9. The fluid dispenser (10) according to any one of claims 1 to 8, further comprising a dispenser housing (12);
wherein the sample preparation mechanism (58) is configured to generate the sample (60) inside the dispenser housing (12); and
wherein the fluid dispenser (10) comprises a sample discharge mechanism for discharging the sample (60) from inside of the dispenser housing (12) to outside of the dispenser housing (12).

10. The fluid dispenser (10) according to any one of claims 1 to 9, further comprising a storage container (132) that is configured to store the sample (60);
wherein the storage container (132) is accessible from outside of the fluid dispenser (10) for collection of the sample (60).

11. The fluid dispenser (10) according to any one of claims 1 to 10, wherein the fluid dispenser (10) is configured to label the sample (60).

12. The fluid dispenser (10) according to any one of claims 1 to 11, further comprising a symptom detection mechanism;
wherein the symptom detection mechanism is configured to detect at least one symptom of a possible infection in a user of the fluid dispenser (10); and
wherein the sample preparation mechanism (58) is configured to generate the sample (60) if the symptom detection mechanism detects that the user has the at least one symptom.

13. The fluid dispenser (10) according to any one of claims 1 to 12, further comprising:
a fluid analyzer (56) for analyzing at least some of the fluid (42) collected by the fluid collector (54); and
at least one processor;
wherein the at least one processor is configured to determine whether or not to generate the sample (60) based, at least in part, on an internal analysis of the fluid (42) performed by the fluid analyzer (56).

14. The fluid dispenser (10) according to claim 13, wherein the sample preparation mechanism (58) is able to prepare different sample types adapted for different external analyses;
wherein the at least one processor is configured to determine which sample type to prepare based, at least in part, on the internal analysis of the fluid (42) performed by the fluid analyzer (56).

15. The fluid dispenser (10) according to any one of claims 1 to 14, wherein the fluid dispenser (10) is configured to provide a notification when the sample (60) has been generated; and
wherein the fluid dispenser (10) is configured to transmit the notification to an external laboratory.
